# EUROPEAN PATENT APPLICATION

(11) **EP 4 732 897 A1**
(43) Date of publication of application: **29.04.2026**
(21) Application number: 25210102.7
(22) Date of filing: 21.10.2025
(51) Int. Cl.: A61N 5/10, A61G 13/00

(54) **RADIOTHERAPY COUCH TOP**

(30) Priority: 25.10.2024 GB 202415783
(71) Applicant: Elekta Limited, Crawley, Sussex RH10 9RR (GB)
(72) Inventor: ALEXIS, Henrik, Crawley (GB)
(74) Representative: Thomas, Tomos Daniel

(57) **Abstract**

Disclosed herein is a radiotherapy couch top configured to support a subject thereon and comprising: a first longitudinal portion with a first lateral width; and a second longitudinal portion with a second lateral width, the second lateral width being smaller than the first lateral width, wherein the second longitudinal portion is integrally formed with the first longitudinal portion.

## Description

This disclosure relates to a radiotherapy couch top, and in particular to a radiotherapy couch top with integrally formed first and second longitudinal portions. This disclosure also relates to a patient support apparatus and a radiotherapy device including the radiotherapy couch top.

### Background

Radiotherapy can be described as the use of ionising radiation, such as X-rays, to treat a human or animal body. Radiotherapy is commonly used to treat cancer, for example to treat tumours within the body of a patient or subject. In such treatments, ionising radiation is used to irradiate, and thus destroy or damage, cells which form part of the tumour.

Tumours can be present in various different anatomical locations within a subject. Treatment plans can be generated and optimised to define how particular subjects with particular tumours will be treated, within the confines of what is possible using the available radiotherapy device. Typically, a the subject is disposed on a patient support apparatus and a source of radiation coupled to a rotatable gantry directs a beam of radiation through the subject from various different angles in order to build up the applied dose at the location of the tumour and limit the applied dose at other anatomical locations within the subject. The subject can be positioned on the patient support apparatus in various positions or poses, including with them lying either on their back or front, with either their head or feet closer to the source of radiation, with their arms either above their head or by their sides, etc. The different angles of irradiation and the the different poses of the subject, among other things, provide degrees of freedom which can be utilised to optimise irradiation of the tumour and limit irradiation of other tissue.

A patient support apparatus can comprise a support base and a couch top which is supported by the support base and which the subject is disposed on. The couch top is generally a rectangular planar structure taking the form of a board that the subject lies on. If it is desired to accommodate the different poses of subjects as described above, extensions are typically provided which can couple to the couch top. Such extensions may be shaped and sized so as to support different parts of the anatomy of subjects and may be coupled to and decoupled from the couch top as needed for different treatments and different subjects.

Imaging can be performed to determine a position of a subject and of anatomical features within the subject. Such imaging can be performed before radiotherapy treatment begins with the subject in a treatment position, and/or can be performed while radiotherapy treatment is occurring. This helps ensure that the radiation is directed at the intended location, i.e. the location of the tumour, and not directed at other locations, e.g. locations of organs at risk. Radiotherapy treatment can be paused or adjusted if it is determined that the tumour is receiving or will receive a lower dose than would be desirable and/or if it is determined that healthy tissue is receiving or will receive a higher dose than would be desirable. By way of example, kV imaging (or MV imaging) can be used to provide images for these purposes.

It is desirable for the images produced by the kV imaging to be of high quality/high accuracy and to have as few artefacts as possible. It is also desirable for such images to accurately cover all relevant parts of the subject within a region of interest. This helps ensure that determinations made regarding locations of anatomical features of the subject, and the doses these anatomical features receive, are as accurate as possible. However, a subject is typically disposed on the couch top during such imaging and during such radiotherapy treatment. In order to image the subject, the imaging beam typically passes through the couch top as well as through the subject. Properties of the couch top can therefore introduce unwanted features into the images or otherwise distort the images, which limits the accuracy with which the subject themselves can be imaged.

In addition, the treatment beam also typically passes through the couch top, which attenuates the dose received by the subject. Efforts can be made to take account of this attenuation in treatment plans, for example through increasing the dose applied from a specific angle if it is known that some of this dose will be attenuated due to the treatment beam passing through the couch top. However, there is a risk of small positioning errors being the difference between unattenuated irradiation of the subject and attenuated irradiation of the subject through the couch top, which could lead to dosimetric errors in the irradiation of the subject.

In view of the above, it would be desirable to increase the accuracy of imaging supporting radiotherapy treatment, as well as to increase the accuracy of such radiotherapy treatment itself.

The present invention seeks to address these and other disadvantages encountered in the prior art.

### Summary

An invention is defined in the independent claims. Optional features are set out in the dependent claims.

### Figures

Specific embodiments are now described, by way of example only, with reference to the drawings, in which:
Figure 1 depicts a radiotherapy device or apparatus according to the present disclosure;
Figure 2 depicts a patient support apparatus according to the present disclosure;
Figure 3 depicts a top-down view of a couch top according to the present disclosure;
Figure 4 depicts a chamfered edge of a couch top according to the present disclosure;
Figure 5 depicts a shoulder angle of a couch top according to the present disclosure;
Figure 6 depicts a transition angle of a couch top according to the present disclosure.

### Detailed Description

In overview, and without limitation, the application relates to a couch top for supporting a subject. The couch top can be implemented as part of a patient support apparatus and can be used to support the subject within a radiotherapy device. The couch top includes a first portion and a second portion at different points along the length of the couch top, with the second portion being narrower than the first portion. In other words, the couch top includes a wider first portion, which can be used to support a torso of a subject, and a narrower second portion, which can be used to support a head of the subject. The first portion and the second portion are integrally formed. In other words, the narrower second portion is not selectively connectable to and separable from the wider first portion, but instead the couch top including the first and second portions is one monolithic, indivisible unit.

The narrower second portion can be used to support a head of a subject without large amounts of couch top material extending either side of the head of the subject. This enables a treatment beam direct access to the head of the subject, without also passing through the couch top, from a greater range of angles, which would otherwise attenuate the treatment beam. This is of particular importance for brain treatments since it is particularly important to be accurate for these treatments. The implementation of the 'omega-shape' of the couch top may prevent limitations to reachable treatment zones that may otherwise apply when using couch kicks/couch rotations, and avoid corners of a rectangular couch top colliding with imaging arms or the treatment head.

For couch tops which are coupled to extensions, a junction is created between the main body of the couch top and the extension. Since the junction causes a discontinuity in the couch top, irradiating through this discontinuity is often avoided since it leads to uncertainty in the attenuated dose actually received by different anatomical locations within the subject disposed on the couch top. In view of the complexity involved in creating a treatment plan accommodating this junction and the safety analysis required when evaluating dose to subjects, clinicians may avoid applying radiation through or close to this junction, thereby reducing the available treatment zones. In addition, kV images can be distorted in the region of the discontinuity, reducing their accuracy or making them unusable in this region and thereby also reducing the available imaging zones.

In contrast, the integrally formed wider first portion and narrower second portion as described herein maximise the treatment and imaging zones through providing a couch top which causes less distortion and attenuation, or at least which causes distortion and attenuation in a predictable, continuous manner which can be accommodated and mitigated more easily and more accurately. The impact of any small positioning errors that may be present on the treatment and the imaging are therefore reduced. The form of the couch top as described herein has been designed to optimize the treatment zone in view of typical body sizes, regions of interest, positioning devices and treatment poses.

The present disclose also provides particular adaptations of a couch top, in particular relating to the angles formed by different surfaces of the couch top, in order to further mitigate the impact of such small positioning errors on the treatment and imaging performed. This further increases the accuracy of such imaging and such image-guided treatment through optimizing attenuation gradients of the beams.

Accordingly, disclosed herein is a radiotherapy couch top configured to support a subject thereon and comprising: a first longitudinal portion with a first lateral width; and a second longitudinal portion with a second lateral width, the second lateral width being smaller than the first lateral width, wherein the second longitudinal portion is integrally formed with the first longitudinal portion.

As discussed herein, these features enable more accurate radiotherapy to be performed through increasing the range of angles from which a head of the subject can be directly irradiated and avoiding discontinuities in the treatment field. Moreover, the avoidance of such discontinuities enables more accurate imaging to be performed. This in turn can further increase the accuracy of radiotherapy treatment informed by or guided by such imaging.

Optionally, the first longitudinal portion of the radiotherapy couch top is configured to support a head of the subject. Optionally, the second longitudinal portion of the radiotherapy couch top is configured to support a torso of the patient.

Optionally, the radiotherapy couch top comprises first and second lateral edges, wherein each of the first and second lateral edges is chamfered. Optionally, a chamfer angle of each of the first and second lateral edges relative to a lower surface of the radiotherapy couch top is is approximately 45°. Optionally, a chamfer angle of each of the first and second lateral edges relative to a lower surface of the radiotherapy couch top is less than 45°.

Optionally, the radiotherapy couch top comprises a third longitudinal portion of the radiotherapy couch top longitudinally between the first longitudinal portion and the second longitudinal portion, wherein the first longitudinal portion, the second longitudinal portion and the third longitudinal portion are all integrally formed with each other. Optionally, the third longitudinal portion has a third lateral width which decreases from being equal to the first lateral width at an end of the third longitudinal portion which is adjacent to the first portion, to being equal to the second lateral width at an end of the third longitudinal portion which is adjacent to the second longitudinal portion.

Optionally, a shoulder angle between a lateral edge of the radiotherapy couch top in the second longitudinal portion and a lateral edge of the radiotherapy couch top in the third longitudinal portion is an acute angle. Optionally, the shoulder angle is smaller than or equal to 70°. Optionally, the shoulder angle is approximately 45°. Optionally, the shoulder angle is smaller than 45°.

Optionally, a transition angle between a lower surface of the first longitudinal portion and a lower surface of the third longitudinal portion is an acute angle. Optionally, the transition angle is at least 10°. Optionally, the transition angle is less than or equal to 45°.

Optionally, the first longitudinal portion is not separable from the second longitudinal portion. Optionally, the radiotherapy couch top comprises one or more fibres extending between the first and second longitudinal portions.

Optionally, a first thickness of the radiotherapy couch top in the first longitudinal region is larger than a second thickness of the radiotherapy couch top in the second longitudinal region. Optionally, a thickness of the radiotherapy couch top gradually decreases between the first longitudinal region and the second longitudinal region. Optionally, the gradual decrease in thickness comprises a lower surface of the radiotherapy couch top between the first longitudinal region and the second longitudinal region having a slope in the range of 10° to 45° relative to a lower surface of the radiotherapy couch top in the first longitudinal region.

Also disclosed herein is a patient support apparatus comprising: the radiotherapy couch top as described above; and a patient support base configured to support the radiotherapy couch top.

Also disclosed herein is a radiotherapy device comprising: a rotatable gantry; a radiation source; an imaging apparatus; and the patient support apparatus as described above.

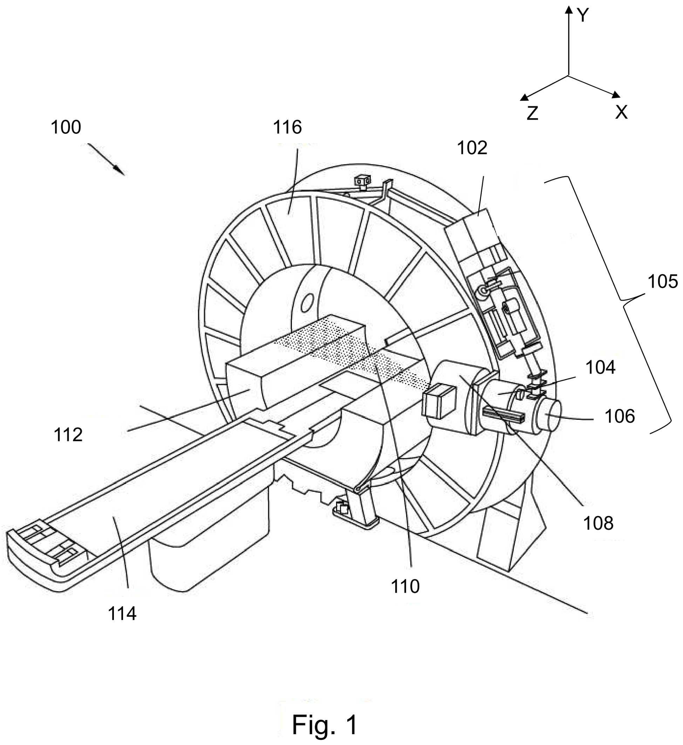

Figure 1 depicts a radiotherapy device 100. The radiotherapy device 100 is suitable for delivering, and configured to deliver, a beam of radiation 110 to a patient during radiotherapy treatment. The device 100 and its constituent components will be described generally for the purpose of providing useful accompanying information for the present application. The device 100 depicted in Figure 1 is in accordance with the present disclosure and is suitable for use with the disclosed systems and apparatuses. While the device 100 in Figure 1 is an MR-linac, in implementations of the present disclosure the device 100 may be another type of radiotherapy device, for example a linac device with a different imaging capability.

In overview, the radiotherapy device 100 comprises a source of radiation 105 and an imaging apparatus 112. The source of radiation 105 is coupled to a rotatable gantry 116. The device 100 comprises a patient positioning apparatus which comprises a patient positioning surface 114 on which a patient may be positioned. Before treatment, the patient is positioned on the surface 114, and the patient positioning apparatus may be used to position the patient in an appropriate position for the treatment, for example according to a reference image on which the patient's treatment plan is based. During treatment, the source of radiation 105 delivers radiation to the patient according to the patient's treatment plan.

The source of radiation 105 is configured to generate a beam of radiation 110, and in particular a beam of therapeutic radiation. The radiation source 105 is attached to the rotatable gantry 116 so as to rotate with the gantry 116. In this way, the radiation source is rotatable around the patient so that the beam 110 can be applied from different angles around the gantry 116. The source of radiation 105 may comprise a beam generation system comprising a linear accelerator (linac). For such a linac device, the beam generation system may comprise a source of RF energy 102, an electron gun 106, and a waveguide 104.

The source 102 of radiofrequency waves, such as a magnetron, is configured to produce radiofrequency waves. The source 102 of radiofrequency waves is coupled to the waveguide 104, for example via a circulator, and is configured to pulse radiofrequency waves into the waveguide 104. Radiofrequency waves may pass from the source 102 of radiofrequency waves through an RF input window and into an RF input connecting pipe or tube. A source of electrons 106, such as an electron gun, is also coupled to the waveguide 104 and is configured to inject electrons into the waveguide 104. In the electron gun 106, electrons are thermionically emitted from a cathode filament as the filament is heated. The temperature of the filament controls the number of electrons injected. The injection of electrons into the waveguide 104 is synchronised with the pumping of the radiofrequency waves into the waveguide 104. The design and operation of the radiofrequency wave source 102, electron source 106 and the waveguide 104 is such that the radiofrequency waves accelerate the electrons to very high energies as the electrons propagate through the waveguide 104.

The design of the waveguide 104 depends on whether the linac accelerates the electrons using a standing wave or travelling wave, though the waveguide 104 typically comprises a series of cells or cavities, each cavity connected by a hole or 'iris' through which the electron beam may pass. The cavities are coupled in order that a suitable electric field pattern is produced which accelerates electrons propagating through the waveguide 104. As the electrons are accelerated in the waveguide 104, the electron beam path is controlled by a suitable arrangement of steering magnets, or steering coils, which surround the waveguide 104. The arrangement of steering magnets may comprise, for example, two sets of quadrupole magnets.

To ensure that propagation of the electrons is not impeded as the electron beam travels toward the target, the waveguide 104 is evacuated using a vacuum system comprising a vacuum pump or an arrangement of vacuum pumps. The pump system is capable of producing ultra-high vacuum (UHV) conditions in the waveguide 104 and in the flight tube. The vacuum system also ensures UHV conditions in the electron gun 106. Electrons can be accelerated to speeds approaching the speed of light in the evacuated waveguide 104.

Once the electrons have been accelerated, they travel toward a heavy metal target which, when impacted by the electrons, generates a beam of high energy photons, forming a radiation beam 110. When the electrons strike the target, X-rays are produced in a variety of directions. The device 100 comprises collimation apparatus 108. The collimation apparatus 108 may comprise a primary collimator. The primary collimator is configured to block X-rays travelling in certain directions and pass only forward travelling X-rays to produce a treatment beam 110. The X-rays may be filtered and may pass through one or more ion chambers for dose measuring. The collimation apparatus 108 may additionally comprise beam shaping apparatus such as a multi-leaf collimator (MLC). The beam can be shaped in various ways by the beam-shaping apparatus. The source of radiation is configured to direct the beam 110 of therapeutic radiation, having been appropriately filtered and shaped by the collimation apparatus 108, toward a patient positioned on the patient support surface 114.

The device 100 comprises an imaging apparatus 112 or 'image acquisition apparatus'. The depicted imaging apparatus 112 is an MR imaging apparatus, though the imaging apparatus may take other forms, for example a cone beam computed tomography (CBCT) apparatus. The MR imaging apparatus 112 is shown in cross-section in the diagram. The imaging apparatus 112 is configured to generate imaging data. The imaging data may comprise images of the patient. The imaging apparatus 112 is therefore configured to obtain images of a patient positioned on the patient support surface 114. The imaging data generated by the imaging apparatus 112 may be used to generate a reference image to enable treatment planning, and/or may be used during the delivery of therapeutic radiation to help guide the beam of radiation 110 or to provide an input into motion management and real-time adaptive radiotherapy techniques.

As described herein, the radiotherapy device 100 may comprise an imaging apparatus 112 which is a kV imaging apparatus. While the skilled person will be familiar with kV imaging apparatuses, the following brief description is provided. The kV imaging apparatus may comprise a kV beam source and a kV detector, each of which may be fixed to the rotatable gantry opposite (at 180° to) each other. Each of the kV beam source and the kV detector may be offset by 90° relative to the source of radiation 105. The kV beam source may be configured to generate a kV X-ray beam directed through the subject (and in general through the patient support surface 114), to be incident on the kV detector. The kV detector may be configured to generate an image of the subject based on the kV X-ray beam it receives, as attenuated by the subject (and in general the patient support surface 114) which the kV X-ray beam passes through.

Figure 1 and its accompanying description herein is provided to give context to the application and to facilitate understanding of the present invention. In addition to the components described in overview above, a radiotherapy device also comprises many other functions, components and subsystems as will be understood by the skilled person.

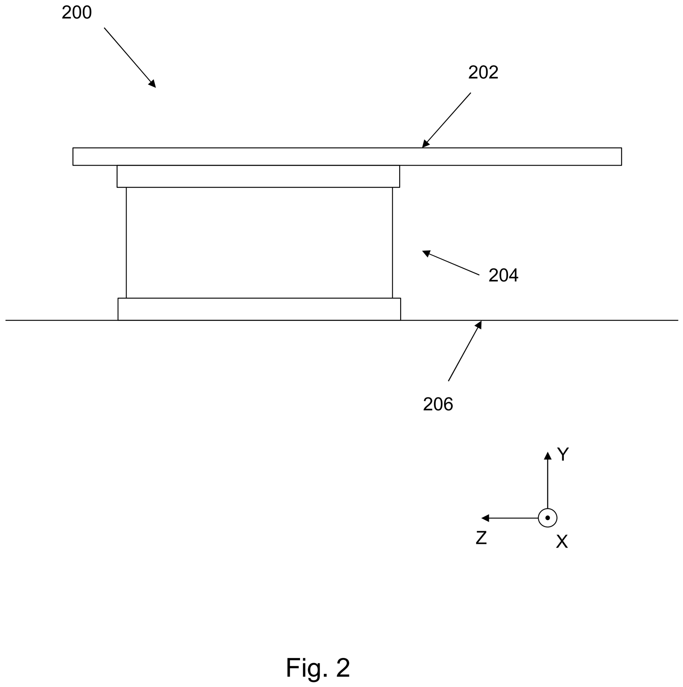

Figure 2 depicts a schematic of a patient support apparatus 200 according to the present disclosure.

The patient support apparatus 200 comprises a couch top 202, i.e. a radiotherapy couch top 202. The patient support apparatus 200, or the couch top 202, may correspond to the patient support apparatus 114 described in relation to Figure 1. The couch top 202 is configured to support a subject, i.e. a patient, thereon. In other words, in use, a subject may be disposed on and in contact with an upper surface of the couch top 202. The couch top 202 may be stiff/rigid enough such that it does not bend or sag significantly when a subject is disposed thereon. The couch top may comprise a foam core with a fibre skin. The fibre skin can comprise, by way of example, carbon fibres or aramid fibres. Such fibres may exhibit adequate strength for supporting the subject while having low atomic numbers, which limits the effect of the fibres on image quality when a kV imaging beam passes through the couch top 202. While the couch top 202 is depicted schematically as being generally flat/linear in the side-on view of Figure 2 for ease of illustration, the present disclosure provides that a couch top 202 has first and second longitudinal portions with first and second lateral widths respectively.

The patient support apparatus 200 also comprises a patient support base 204. The patient support base 204 is configured to support the couch top 202. The patient support base 204 may be disposed on a floor 206 of a room housing the patient support apparatus 200/radiotherapy device 100. The patient support base 204 may comprise one or more drivers, actuators and/or other mechanical/electrical components configured to cause movement of the couch top 202. This movement may comprise linear movement along three perpendicular axes, as well as rotational movement around each of these three perpendicular axes. In other words, the patient support apparatus 200 may be configured for 6D movement, though in some examples the patient support apparatus 200 may not be configured to provide movement along/around one or more of these directions, e.g. may be configured for 3D, 4D or 5D movement.

The couch top 202 may be fixed to the patient support base 204 such that tilting or translation of all or part of the patient support base 204 causes tilting or translation of the couch top 202. This may be used to position the couch top 202, and the subject thereon, into a desired position for imaging and/or radiotherapy. The couch top 202 may be slidably coupled to the patient support base 204, enabling linear movement of the couch top 202 relative to the patient support base 204 (e.g. along the Z-axis as depicted in Figure 2). In some examples, some of the driving electronics or mechanical features may be couped to or integrated into the couch top 202, in a region of the couch top 202 (an 'attachment zone') which is not irradiated by treatment or imaging beams. One or more attachment blocks may be provided (in the attachment zone) between the couch top 202 and the patient support base 204. For example, four such attachment blocks may be provided. The one or more attachment blocks may be configured to couple the couch top 202 to the patient support base 204.

The patient support base 204 may at least be configured to cause the couch top to translate along a left-right direction as depicted in Figure 2 (parallel to the Z-axis). Other components of the radiotherapy apparatus 100 of Figure 1, such as the source of radiation 105 and the imaging apparatus 112, may generally be located to the right of the patient support apparatus 200 as depicted in Figure 2. In a 'setup position' which enables a subject to easily mount the couch top 202, the couch top 202 may be translated/disposed towards the left of Figure 2. The couch top 202 may be translated/disposed to the right as depicted in Figure 2, i.e. towards the other components of the radiotherapy apparatus 100, to place the subject in a 'treatment position' in which they can be imaged and/or treated. The couch top 202 may for example be translated in this manner using a linear guide or linear rail of the patient support base 204 coupled to the couch top 202.

The present application focuses on the form of the couch top 202 as described herein. It will be appreciated that this couch top 202 is generally applicable to various different forms of patient support base 204 and patient support apparatus 200 without limitation, and that the depiction and discussion of Figure 2 is provided merely as an example for understanding the context of the current application. The couch top 202 is the part of the patient support apparatus 200 disposed to contact the subject and configured for support of the subject, in contrast to the parts of the patient support apparatus 200 configured to control/drive movement of the couch top 202 with the subject thereon.

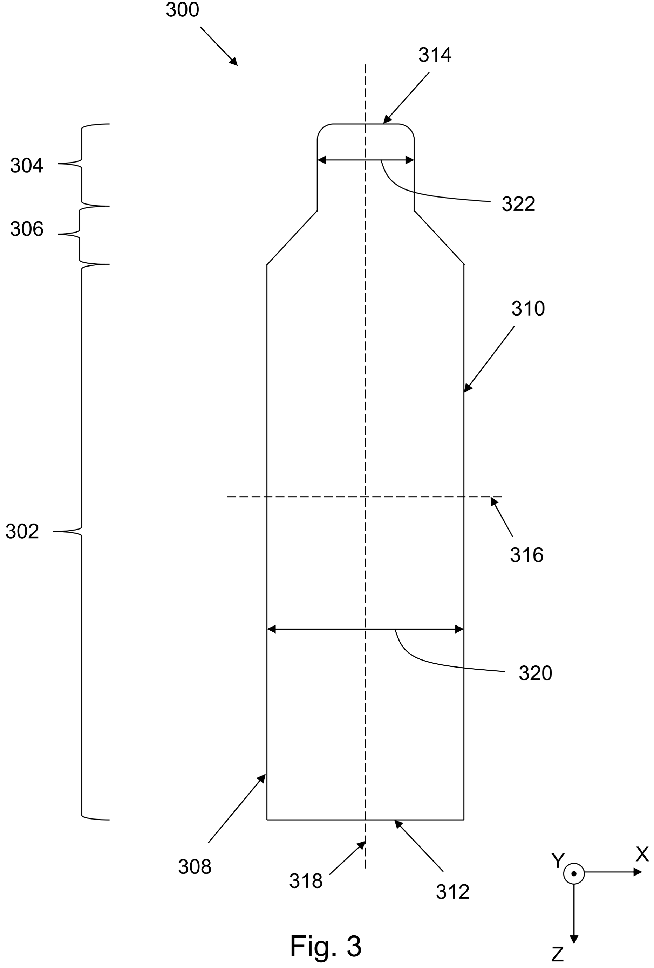

Figure 3 depicts a top-down view of a couch top 300 according to the present disclosure. The couch top 300 may correspond to the couch top 202 described in relation to Figure 2.

The couch top 300 comprises a first longitudinal portion 302 and a second longitudinal portion 304. In other words, the couch top 300 comprises a first portion in a first longitudinal region 302 of the couch top 300 and a second portion in a second longitudinal region 304 of the couch top 300. The couch top 300 may also comprise a third longitudinal portion 306, i.e. a third portion in a third longitudinal region 306 of the couch top, between the first longitudinal portion 302 and the second longitudinal portion 304.

The couch top 300 may comprise a first lateral side 308 and a second lateral side 310. The couch top 300 may comprise a first longitudinal end 312 and a second longitudinal end 314. The couch top 300 may extend between the first lateral side 308 and the second lateral side 310, each of which may be described as being disposed at different ends of a lateral axis 316 of the couch top 300. The couch top 300 may extend between the first longitudinal end 312 and the second longitudinal end 314, each of which may be described as being disposed at different ends of a longitudinal axis 318 of the couch top 300. The longitudinal axis 318 may be perpendicular to the lateral axis 306. The first and second lateral sides 308, 310 may be parallel to the longitudinal axis 318. The first and second longitudinal ends 312, 314 may be parallel to the lateral axis 316.

The first longitudinal portion 302 of the couch top 300 has a first lateral width 320. This lateral width is a width of the couch top 300 along the lateral axis 316 of the couch top 300 between the first lateral side 308 and the second lateral side 310. The couch top 300 may have the first lateral width 320 throughout or in most of the first longitudinal portion 302. The second longitudinal portion 304 of the couch top 300 has a second lateral width 322. This lateral width is a width of the couch top 300 along the lateral axis 316 of the couch top 300 between the first lateral side 308 and the second lateral side 310. The couch top 300 may have the second lateral width 322 throughout or in most of the second longitudinal portion 304.

The second lateral width 322 of the second longitudinal portion 304 is smaller than the first lateral width 320 of the first longitudinal portion 302. In other words, the first longitudinal portion 302 is wider than the second longitudinal portion 304. The second longitudinal portion 304 is narrower than the first longitudinal portion 302. The couch top 300 is not of uniform width along its length (along the longitudinal axis 318 thereof), but instead has a wider portion along a first part of its length and a narrower portion along a second part of its length.

The first longitudinal portion 302 is at or adjacent to the first longitudinal end 312. The first longitudinal portion 302 is disposed closer to the first longitudinal end 312 than the second longitudinal portion 304 is. The first longitudinal portion 302 may comprise an attachment zone configured to couple the couch top 300 to the patient support base 204 of the patient support apparatus 200 (see Figure 2). The attachment zone may be disposed adjacent to the first longitudinal end 312, i.e. at the bottom of Figure 3. The first longitudinal portion 302 may comprise a body treatment zone configured to support a torso of a subject disposed on the couch top 300. The body treatment zone may be disposed closer to the second longitudinal portion 304 than the attachment zone is. The body treatment zone may be disposed between the attachment zone and the second longitudinal portion 304 or between the attachment zone and the third longitudinal portion 306. In use, the couch top 300 may be positioned in relation to other components of the radiotherapy device 100 such that imaging and/or therapeutic radiation passes through the body treatment zone but not through the attachment zone.

The second longitudinal portion 304 is at or adjacent to the second longitudinal end 314. The second longitudinal portion 304 may be comprise or correspond to a head treatment zone configured to support a head of a subject disposed on the couch top 300. In use, imaging and/or therapeutic radiation passes through the head treatment zone. In view of the relative sizes of human torsos and heads, the second longitudinal portion 304 may be shorter (along the longitudinal axis 318) than the first longitudinal portion 302 and is narrower (along the lateral axis 316) than the first longitudinal portion 302.

Since the second longitudinal portion 304 has the narrower second lateral width 322, the head of the subject is more exposed in that a radiotherapy beam can be directed through the head of the subject, without also having to pass through the couch top 300, from a greater range of positions/angles of the source of radiation 105. This may increase the accuracy, efficiency and/or versatility of head treatments. Typically, radiotherapy is applied with the source of radiation 105 located at various angles around the subject using the rotatable gantry 116. It will be appreciated that, if the couch top 300 had the same, first longitudinal width along its whole length, the radiotherapy beam would have to pass through the couch top 300 for irradiation with the source of radiation 105 positioned in at least the lower 180° of the circle described by the rotatable gantry 116. However, since the second longitudinal region 304 is provided with the smaller, second lateral width 322, depending on the particular anatomical target location within the head, this enables direct irradiation of the target without the radiotherapy beam also passing through the couch top 300 with the source of radiation 105 at a greater range of angles. For example, this may be enabled with the source of radiation 105 substantially to the left or right of the couch top 300 (along the X-axis), but below the height of the couch top 300 to some extent (along the Y-axis). This increases the scope for generating treatment plans and corresponding treatments which more accurately target particular locations and shapes of tumours. Moreover, the narrower second lateral width 322 may prevent the kV imaging detector/panel from colliding with the couch top when it rotates.

The above discussion has focused on the subject being reclined with their head disposed in the second longitudinal portion 304 and their torso disposed in the first longitudinal portion 302. The subject may be disposed on their back with their back in contact with an upper surface of the couch top 300. Alternatively, the subject may be disposed on their front with their front in contact with the upper surface of the couch top 300. Moreover, in some examples the subject may be disposed on the couch top in other ways. For example, in 'feet first' treatments, the subject may be disposed with their head adjacent to the first longitudinal end 312 and their feet adjacent to the second longitudinal end 314.

An extension or cover board configured to couple to the second longitudinal portion 304 of the couch top 300 may be provided. The extension may have a lateral width equal or similar to the first lateral width 320 of the first longitudinal portion 302 of the couch top 300. The extension may be used for better supporting the feet/legs of the subject in feet first treatments and may be used for better supporting the arms of the subject in some treatments in which the subject is lying on their front.

The third longitudinal portion 306 may couple the first longitudinal portion 302 to the second longitudinal portion 304. The third longitudinal portion 306 may have a third lateral width which varies along its length. The third lateral width may be equal to the first lateral width 320 of the first longitudinal portion 302 at an end of the third longitudinal portion 306 that is adjacent to the first longitudinal portion 302. The third lateral width may be equal to the second lateral width 322 of the second longitudinal portion 304 at an end of the third longitudinal portion 306 that is adjacent to the second longitudinal portion 304.

In other words, the third lateral width may decrease along the longitudinal axis 318 of the couch top 300 when moving towards the second longitudinal end 314 and away from the first longitudinal end 312. The rate of this decrease in width may be substantially constant. In other words, the first and second lateral sides 308, 310 of the couch top in the third longitudinal portion 306 may be straight lines. As further described herein, the first and second lateral sides 308 of the couch top in the third longitudinal portion 306 may be (diagonally) angled with respect to the longitudinal axis 318, e.g. may form an approximately 45° angle with the longitudinal axis 318.

The third longitudinal portion 306 may provide a gradual transition between the first longitudinal region 302 and the second longitudinal region 304. Considering the dose applied to a subject in a radiotherapy treatment, whether or not the radiotherapy beam passes through the couch top 300 or not can significantly affect the dose received by the subject. As such, when there is a sharp cut off between presence and absence of the couch top 300, small positional errors bridging the cut off can lead to large dosimetric errors. As further described herein, the gradual transition provided by the third longitudinal portion 306 can reduce the magnitude of such dosimetric errors resulting from small positional errors.

The first longitudinal portion 302 is integrally formed with the second longitudinal portion 304. In examples in which the third longitudinal portion 306 is present, this may be achieved by the first longitudinal portion 302 being integrally formed with the third longitudinal portion 306 and the third longitudinal portion 306 being integrally formed with the second longitudinal portion 304, such that the first and second longitudinal portions 302, 304 are ultimately integrally formed. The different portions of the couch top 300 may be described as being integrally formed, being formed as one structure, being monolithic, and/or being formed without joints therebetween. As described herein, this can help prevent such joints distorting the images produced of the subject and/or can help prevent dosimetric discrepancies.

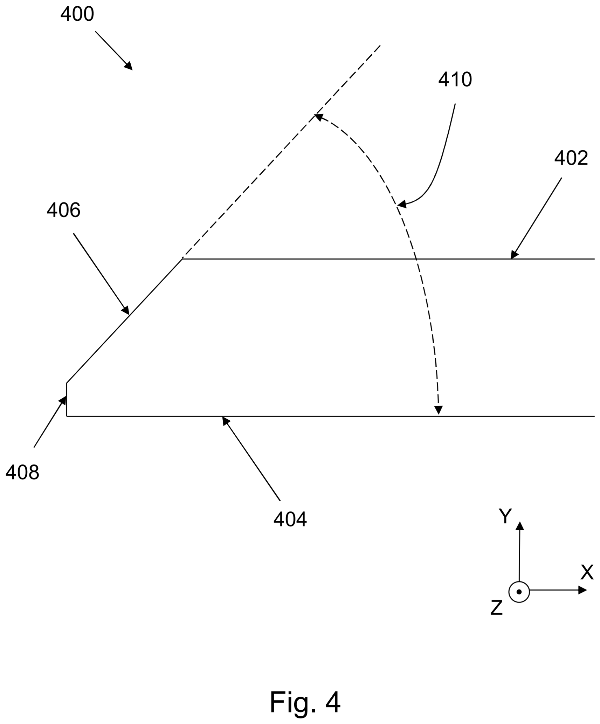

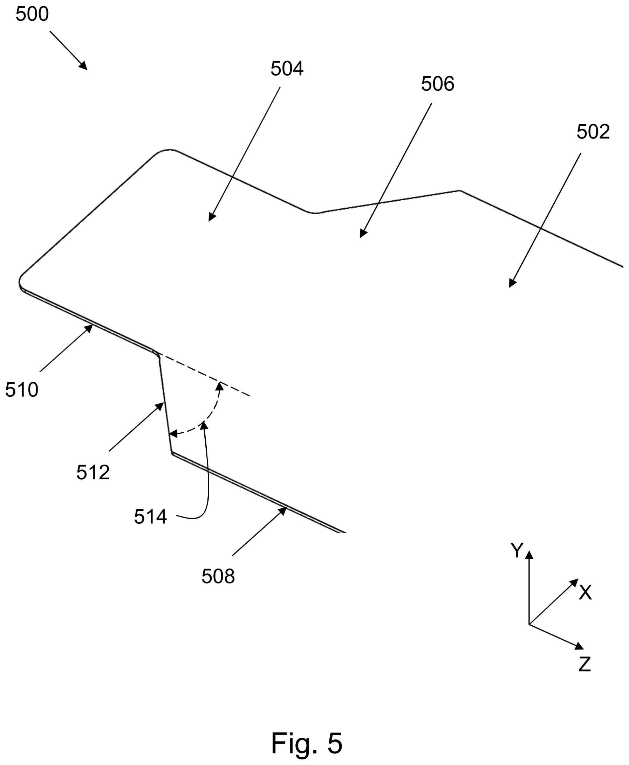

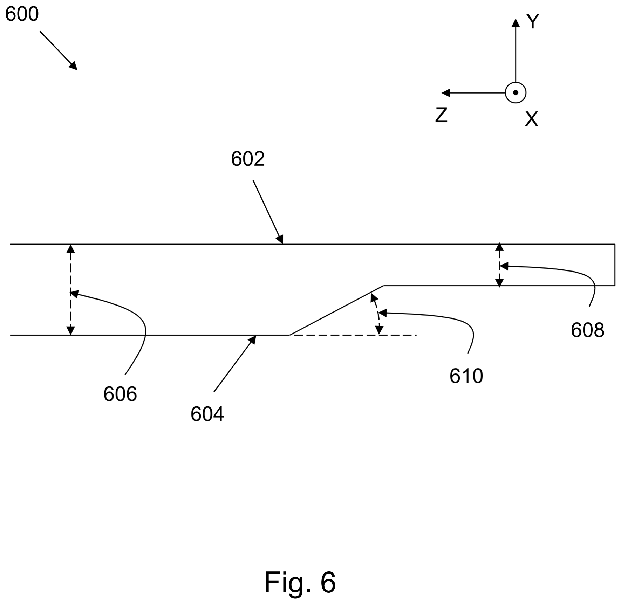

In addition, the present disclosure provides various further features of the couch top 300 which can further increase the reliability and accuracy of imaging and radiotherapy treatment. In particular, as described in relation to Figures 4-6 below, various angles of the couch top 300 can be optimised to further improve the imaging and radiotherapy treatment that is achievable. A chamfer angle (in the X-Y plane of the couch top 300) will be discussed in relation to Figure 4, a shoulder angle (in the X-Z plane of the couch top 300) will be discussed in relation to Figure 5, and a transition angle (in the Y-Z plane of the couch top 300) will be discussed in relation to Figure 6. The radiotherapy couch tops described herein may comprise any one, multiple or all of these in combination.

Figure 4 depicts a chamfered edge of a couch top 400 according to the present disclosure. The couch top 400 depicted in Figure 4 may correspond to the couch tops 202 and 300 described in relation to Figures 2 and 3 respectively. Figure 4 depicts a cross-section of a lateral edge of the couch top 400, and this lateral edge may correspond to one or both of the lateral edges 308, 310 described in relation to Figure 3. In particular, Figure 4 may depict the first lateral edge 308, wherein the second lateral edge 310 can have substantially the same form as the first lateral edge 308 except that it is reversed/flipped with respect to the Y-axis as depicted in Figure 4. The cross-section of Figure 4 is in a plane of the couch top 400 parallel to the lateral axis 316 of the couch top 400 at a point along the longitudinal axis 318 of the couch top 400 (see Figure 3). As depicted in Figure 4, physical features of the couch top 400 are depicted using solid lines, geometrical properties are depicted using dashed lines, and reference numerals are linked to the features they represent using lines incorporating arrows.

The couch top 400 comprises an upper surface 402 and a lower surface 404. In use, a subject may be disposed on and in contact with the upper surface 402 of the couch top 400. As depicted in Figure 4, the upper surface 402 may be flat or planar and the lower surface 404 may be flat or planar. The lower surface 404 may be parallel to the upper surface 402. In other examples, the lower surface 404 and/or the upper surface 402 may be curved (such that a lateral centre of these surfaces is at a lower vertical position than the lateral sides of these surfaces).

The couch top 400 may comprise a chamfered edge 406. In other words, the lateral edge between the upper surface 402 and the lower surface 404 may comprise a surface which is diagonally, rather than perpendicularly, orientated with respect to the upper surface 402 and the lower surface 404. The chamfered edge may also be referred to as a chamfer, a bevel, a chamfered surface or a bevelled surface. The lateral edge may also comprise a perpendicular surface 408 which is disposed perpendicular to the upper surface 402 and the lower surface 404, i.e. is disposed parallel to the Y-axis. Moving around the lateral edge of the couch top 400, the upper edge 402 may be connected to the chamfered edge 406, the chamfered edge 406 may be connected to the perpendicular surface 408, and the perpendicular surface 408 may be connected to the lower surface 404. The chamfered edge 406 may comprise an angled/transitional surface between the upper surface 402 and the perpendicular surface 408.

As depicted in Figure 4, the chamfered edge 406 may form a chamfer angle 410 with the lower surface 404, this chamfer angle 410 being an acute angle. In other words, an angle formed by extending the line described by the chamfered edge 406 and extending the line described by the lower surface 404 such that they intersect would cause these to intersect at a chamfer angle 410 which is an acute angle. Since, as depicted in Figure 4, the upper surface 402 is parallel to the lower surface 404, the chamfer angle 410 may be defined in a corresponding manner relative to the upper surface 402, i.e. the chamfer angle 410 between the chamfered edge 6 and the upper surface 410 may be an acute angle. As depicted in Figure 4, the chamfer angle 410 and the surface formed by the chamfered edge 406 are defined in the X-Y plane of the couch top 400.

The chamfered edge 406 provides that there is a less sharp cut-off relative to couch tops in which an entirety of a lateral edge joining upper and lower surfaces is perpendicular to the upper and lower surfaces. In such couch tops, there is an immediate transition (when moving along the X-axis) between there being no couch top present to there being a substantial, constant thickness of couch top present. When there is a small positioning error or a small deviation of a feature within manufacturing tolerances, such a discontinuity can have a substantial effect on how much an imaging beam or a treatment beam is attenuated as a result. In other words, if the treatment beam is at or close to the edge of the couch top 400, a small misposition will have a large impact on the dose delivered to the subject. Therefore, the chamfered edge 406 reduces the sensitivity of treatment to positioning errors and deviations within manufacturing tolerances by slowly phasing in material in the treatment field.

As described above, the chamfer angle 410 is an acute angle. It is desirable that the chamfer angle be relatively small, such as less than or equal to 45°, such that the thickness of the couch top increases only gradually when moving in the X-direction and therefore such that the sensitivity of the dose delivered to the patient to mispositioning is reduced.

In some scenarios, for example some arrangements of radiotherapy devices, it may be possible for a subject or clinician to injure themselves on the lateral edge of the couch top 400. For example, the lateral edge may trap a finger of the subject or clinician between the lateral edge and another surface of the radiotherapy device or a feature of the surrounding room and equipment. This is of particular concern if the lateral edge forms a knife-edge shape in which it is particularly sharp, i.e. the chamfer angle 410 is particularly small. Therefore, in order to both increase the thickness of the couch top only gradually and lower the risk of injury in combination, a chamfer angle of 45° may be particularly advantageous and optimised. The perpendicular surface 408 may also lower the risk of such injuries.

While Figure 4 depicts the lateral edge of the couch top 400 in cross-section, the same form of lateral edge may be present along the whole or most of the lateral edges of the couch top 400 (i.e. along the Z-direction). For example, the same form of lateral edge may be present in the first longitudinal portion 302, the second longitudinal portion 304, and/or the third longitudinal portion 306. In some examples, the chamfer angle 410 may be different in these different longitudinal portions. This may be implemented to reflect a different balance between dosimetric and injury concerns. For example, the chamfer angle 410 may be made smaller in the second longitudinal portion 410 because it may be particularly important to avoid dosimetric errors to a brain of the subject. As referred to above, for the lateral edge 310, the chamfered edge may be the mirror image of that depicted in Figure 4 (relative to the Y-axis).

Figure 5 depicts a shoulder angle of a couch top 500 according to the present disclosure. The couch top 500 depicted in Figure 5 may correspond to the couch tops 202, 300 and 400 described in relation to Figures 2, 3 and 4 respectively. As depicted in Figure 5, physical features of the couch top 500 are depicted using solid lines, geometrical properties are depicted using dashed lines, and reference numerals are linked to the features they represent using lines incorporating arrows.

The couch top 500 comprises a first longitudinal portion 502, a second longitudinal portion 504 and a third longitudinal portion 506 as defined herein. The first longitudinal portion 502 comprises a first lateral edge 508, the second longitudinal portion comprises a second lateral edge 510, and the third longitudinal portion 506 comprises a third lateral edge 512. While only one of each of these lateral edges 508, 510, 512 is labelled in Figure 5, it will be appreciated that corresponding lateral edges are present on the other side of the couch top 500 (in the positive X-direction).

As described herein, the third longitudinal portion 506 decreases in lateral width moving in the negative Z-direction from the first longitudinal portion 502 to the second longitudinal portion 504, and thereby provides a smooth transition between these portions. The decrease in lateral width of the third longitudinal portion 506 is defined herein using a shoulder angle 514 between the second lateral edge 510 of the second longitudinal portion 504 and the third lateral edge 512 of the third longitudinal portion 506. The shoulder angle 514 is defined in the X-Z plane of the couch top 500.

Providing the narrower second longitudinal portion 504 may better conform the shape of the couch top 500 to the shape of the subject and enable direct irradiation of the head of the subject, without attenuation by the couch top 500, from a greater range of angles. The shoulder angle 514 is an acute angle. This shoulder angle 514 entails that, when moving along the Z-axis, material of the couch top 500 enters the treatment field in a gradual manner relative to the situation in which there is a 90° corner between the wider and narrower ends of the couch top 500. This means that small positioning errors or deviations within manufacturing tolerances can lead to less significant variation in the dose applied to the subject, because the lateral width of the couch top 500 is gradually varied along the Z-axis rather than varied via a sharp cut-off or discontinuity. Therefore, the shoulder angle 514 reduces the sensitivity of treatment, and imaging, to positioning errors and deviations within manufacturing tolerances.

In order to provide a gradual transition and thereby lessen the sensitivity to mispositioning, it is desirable for the shoulder angle 514 to be relatively small. However, the smaller the shoulder angle 514, in general the longer the longitudinal length (along the Z-axis) required for the couch top 500 to narrow from the first lateral width to the second lateral width, and the less well the couch top may conform to the shape of the subject. This may in turn restrict the treatment angles available for direct irradiation of the subject, e.g. of the head of the subject. Therefore, in view of simultaneously optimising both of these competing considerations, the shoulder angle 514 may at least be formed to be smaller than or equal to 70°, and particularly advantageously to be approximately 45°.

Figure 6 depicts a transition angle of a couch top 600 according to the present disclosure. The couch top 600 depicted in Figure 6 may correspond to the couch tops 202, 300, 400 and 500 described in relation to Figures 2, 3, 4 and 5 respectively. As depicted in Figure 6, physical features of the couch top 600 are depicted using solid lines, geometrical properties are depicted using dashed lines, and reference numerals are linked to the features they represent using lines incorporating arrows. Figure 6 depicts a side-on view of the couch top 600 in the Y-Z plane.

The couch top 600 comprises an upper surface 602 and a lower surface 604. The left side of Figure 6 may depict part of the first longitudinal portion of the couch top 600 as described herein, and the right side of Figure 6 may depict the second longitudinal portion of the couch top 600 as described herein. The couch top 600 has a first thickness 606 in (at least part of) the first longitudinal portion. The couch top 600 has a second thickness 608 in (at least part of) the second longitudinal portion.

The second thickness 608 is smaller than the first thickness 606. In other words, the couch top 600 is thinner (in the Y-direction) in the second longitudinal portion than in the first longitudinal portion. Since the second longitudinal portion may support a head of the subject, it may be particularly important to be accurate when irradiating this region. The smaller second thickness 608 in this region can reduce the attenuation of the treatment beam (and the imaging beam) and facilitate more accurate treatment. Since the head of the subject is less heavy than the torso of the subject, this may be possible without leading to an unacceptable degree of bending of the couch top 600. In other examples, the couch top 600 may have the same thickness in the first and second longitudinal portions, i.e. the first thickness 606 and the second thickness 608 may be the same.

As depicted in Figure 6, the couch top does not comprise a perpendicular discontinuity between the regions with the first thickness 606 and the second thickness 608. Instead, the thickness of the couch top 600 gradually decreases (when moving in the negative Z-direction). This may be achieved through the lower surface 604 of the couch top 600 not being parallel to the Z-axis/upper surface 602 of the couch top 600 in the region of the couch top 600 in which the transition from the first thickness 606 to the second thickness 608 occurs. The angle at which this transition occurs is defined by a transition angle 610.

In some examples, the whole of the first longitudinal portion may have the first thickness 606, the whole of the second longitudinal portion may have the second thickness 608, and the region in which the thickness transitions between the first thickness 606 and the second thickness 608 may correspond to the third longitudinal portion 608. In other examples, the region in which the thickness transitions between the first thickness 606 and the second thickness 608 may span/extend from part of the first longitudinal portion to part of the third longitudinal portion. In other examples, the region in which the thickness transitions between the first thickness 606 and the second thickness 608 may span/extend from part of the third longitudinal portion to part of the second longitudinal portion. In other examples, the region in which the thickness transitions between the first thickness 606 and the second thickness 608 may be contained within a subset of the third longitudinal portion. In any case, at least part of the first longitudinal region may have the first thickness 606, at least part of the second longitudinal region 608 may have the second thickness 608, and the thickness of the couch top 600 gradually transitions from the first thickness 606 to the second thickness 608 according to the transition angle 610.

The transition angle 610 may be defined between a lower surface of the first longitudinal portion and a lower surface of the third longitudinal portion. The transition angle 610 is an acute angle. The transition angle 610 enables the different thicknesses of the couch top 600 to be provided (as described above), without there being a discontinuity in thickness which could lead to large dosimetric inaccuracies as a result of small positioning errors or deviations within manufacturing tolerances. Therefore, the transition angle 610 further reduces the sensitivity of treatment to positioning errors and deviations within manufacturing tolerances.

The transition angle 610 is desirably relatively small so as to provide a gradual increase in thickness, for example smaller than or equal to 45°. However, the smaller the transition angle 610, the longer the region in which the transition occurs is, which could lead to a thicker than desirable couch top 600 in at least part of the second longitudinal portion. Therefore, in view of this additional consideration, the transition angle may be formed to be at least 10°.

It is to be understood that the above description is intended to be illustrative, and not restrictive. Many other implementations will be apparent to those of skill in the art upon reading and understanding the above description. Although the present disclosure has been described with reference to specific example implementations, it will be recognized that the disclosure is not limited to the implementations described, but can be practiced with modification and alteration within the spirit and scope of the appended claims. Accordingly, the specification and drawings are to be regarded in an illustrative sense rather than a restrictive sense. The scope of the disclosure should, therefore, be determined with reference to the appended claims, along with the full scope of equivalents to which such claims are entitled.

## Claims

1. A radiotherapy couch top configured to support a subject thereon and comprising:
a first longitudinal portion with a first lateral width; and
a second longitudinal portion with a second lateral width, the second lateral width being smaller than the first lateral width,
wherein the second longitudinal portion is integrally formed with the first longitudinal portion.

2. The radiotherapy couch top according to claim 1, wherein the first longitudinal portion of the radiotherapy couch top is configured to support a head of the subject.

3. The radiotherapy couch top according to claim 1 or claim 2, wherein the second longitudinal portion of the radiotherapy couch top is configured to support a torso of the patient.

4. The radiotherapy couch top according to any preceding claim, comprising first and second lateral edges, wherein each of the first and second lateral edges is chamfered,
optionally wherein:
a chamfer angle of each of the first and second lateral edges relative to a lower surface of the radiotherapy couch top is approximately 45°; or
a chamfer angle of each of the first and second lateral edges relative to a lower surface of the radiotherapy couch top is less than 45°.

5. The radiotherapy couch top according to any preceding claim, comprising a third longitudinal portion of the radiotherapy couch top longitudinally between the first longitudinal portion and the second longitudinal portion, wherein the first longitudinal portion, the second longitudinal portion and the third longitudinal portion are all integrally formed with each other,
optionally wherein the third longitudinal portion has a third lateral width which decreases from being equal to the first lateral width at an end of the third longitudinal portion which is adjacent to the first portion, to being equal to the second lateral width at an end of the third longitudinal portion which is adjacent to the second longitudinal portion.

6. The radiotherapy couch top according to claim 5, wherein a shoulder angle between a lateral edge of the radiotherapy couch top in the second longitudinal portion and a lateral edge of the radiotherapy couch top in the third longitudinal portion is an acute angle.

7. The radiotherapy couch top according to claim 6, wherein the shoulder angle is smaller than or equal to 70°.

8. The radiotherapy couch top according to claim 6, wherein the shoulder angle is approximately 45°.

9. The radiotherapy couch top according to claim 6, wherein the shoulder angle is smaller than 45°.

10. The radiotherapy couch top according to any of claims 5-9, wherein a transition angle between a lower surface of the first longitudinal portion and a lower surface of the third longitudinal portion is an acute angle,
optionally wherein:
the transition angle is at least 10°; or
the transition angle is less than or equal to 45°.

11. The radiotherapy couch top according to any preceding claim, wherein the first longitudinal portion is not separable from the second longitudinal portion.

12. The radiotherapy couch top according to any preceding claim, wherein the radiotherapy couch top comprises one or more fibres extending between the first and second longitudinal portions.

13. The radiotherapy couch top according to any preceding claim, wherein a first thickness of the radiotherapy couch top in the first longitudinal region is larger than a second thickness of the radiotherapy couch top in the second longitudinal region,
optionally wherein a thickness of the radiotherapy couch top gradually decreases between the first longitudinal region and the second longitudinal region,
further optionally wherein the gradual decrease in thickness comprises a lower surface of the radiotherapy couch top between the first longitudinal region and the second longitudinal region having a slope in the range of 10° to 45° relative to a lower surface of the radiotherapy couch top in the first longitudinal region.

14. A patient support apparatus comprising:
the radiotherapy couch top of any preceding claim; and
a patient support base configured to support the radiotherapy couch top.

15. A radiotherapy device comprising:
a rotatable gantry;
a radiation source;
an imaging apparatus; and
the patient support apparatus of claim 14.
